## Europäisches Patentamt

## European Patent Office

## Office européen des brevets

(11) Publication number: **0 495 995 A1**

## EUROPEAN PATENT APPLICATION
published in accordance with Art. 158(3) EPC

(21) Application number: 91913830.5

(22) Date of filing: 03.08.91

(86) International application number: **PCT/JP91/01040**

(87) International publication number: **WO 92/02619 (20.02.92 92/05)**

(51) Int. Cl.5: **C12N 15/12**, C12N 15/72, C12N 1/21, C12N 9/99, C12P 21/02, C07K 13/00, A61K 37/64, //(C12N1/21, C12R1:19),(C12P21/02, C12R1:19)

(30) Priority: 03.08.90 JP 205164/90
19.12.90 JP 411594/90

(43) Date of publication of application:
**29.07.92 Bulletin 92/31**

(84) Designated Contracting States:
**AT BE CH DE ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **SUWA, Yorimasa**
**7-24-13, Shinjuku**
**Shinjuku-ku, Tokyo 160(JP)**
Inventor: **IMAIZUMI, Atsushi**
**5-10-8, Nishihirayama**
**Hino-shi, Tokyo 191(JP)**
Inventor: **OKADA, Masahiro**
**3-5-18, Tamadaira**

**Hino-shi, Tokyo 191(JP)**
Inventor: **SUZUKI, Yoji**
**2-24-10, Higashitoyoda**
**Hino-shi, Tokyo 191(JP)**
Inventor: **KUDO, Ichiro**
**1-28-15, Minamisenzoku**
**Ota-ku, Tokyo 145(JP)**
Inventor: **INOUE, Keizo**
**1-3-17-605, Echujima**
**Koto-ku, Tokyo 135(JP)**
Inventor: **AZUMA, Chieko**
**212, Teijin Tama-Apart., 3-18-4, Tamadaira**
**Hino-shi, Tokyo 191(JP)**
Inventor: **MURAKAMI, Makoto**
**105, Flat Hongo, 5-11-14, Hongo, Bunkyo-ku**
**Tokyo 113(JP)**

(74) Representative: **Harrison, Ivor Stanley**
**Withers & Rogers 4 Dyer's Building Holborn**
**London EC1N 2JT(GB)**

(54) PLASMID FOR USE IN PRODUCING INFLAMED REGION DERIVED PHOSPHOLIPASE A 2? INHIBITORY PROTEIN, MICROBIAL CELL, AND PRODUCTION AND USE OF SAID PROTEIN.

(57) A plasmid for use in producing an inflamed region derived phospholipase $A_2$ inhibitory protein; a microbial cell transformed with the plasmid; a process for producing the protein by culturing the cell; and the protein thus produced and an allergic reaction supresor containing it as the active ingredient. The plasmid contains a DNA sequence coding for a rat or human inflamed region derived phospholipase $A_2$ inhibitory protein and a DNA sequence having a promoter function of regulating the expression of the protein. Escherichia coli is mainly used as the host cell.

# Fig.1

Technical Field

The present invention relates to a recombinant plasmid having a DNA fragment encoding inhibitory protein for phospholipase $A_2$ originated from inflammatory sites, a recombinant microbial cell transformed with the plasmid, a method of producing of inhibitory protein for phosphlipase $A_2$ originated from inflammatory sites by an expression of a gene of said protein. Further, the invention relates to a pharmaceutical composition for a suppression of allergic reactions, containing the protein produced, by using the above method, as an active component.

Background Art

It is known that a complement component C3, hereinafter referred to as "C3", is a protein that plays a crucial role in the complemental pathway. C3, however, is degraded by proteinases present in blood, and thus C3 is first cleaved with C3 convertase to produce C3a and C3b. This C3b is bonded to an extraneous substance through a thiol ester site thereof, following by an activation of the complemental pathway, to thereby form a membrane attack complex. C3a, acts as an anaphylatoxin C3b is then cleaved with a proteinase to finally produce C3dg or C3d.

Phospholipase $A_2$ is an enzyme which hydrolyze a bond of a phospholipid to produce a fatty acid and lysophospholipid. In particular, since the enzyme releases arachidonic acid, which can be a precursor of prostaglandins, leukotrienes, thromboxanes, and the like, from a membrane phospholipid, it is believed that the enzyme can play an important role in producing such an inflammation mediator. Recently, a phospholipase $A_2$ was purified from the inflammatory sites in humans and some animals with inflammatory diseases, and characterized. It is believed that phospholipase $A_2$ accelerates the inflammatory reaction, and therefore, it is expected that drugs having a phospholipase $A_2$ inhibitory activity acts as an antiphlogistic.

The present inventors have carried out research on phospholipase $A_2$, and have screened proteins that specifically inhibit activity of phospholiase $A_2$. As a result, the inventors found that a C3dg originated from human and rats has a specific inhibitory activity on phospholipase $A_2$ from inflammatory sites, i.e., C3dg inhibits the phospholipase $A_2$ in the inflammatory sites and can act as an anti-inflammatory drug, and thus, Japanese Patent Application No. 1-200246 and 2-89085 corresponding to WO 91/01999 published after the filing date of Japanese Patent Application No. 2-205164, which claimed, a priority in the present application, were filed by the inventors at the Japanese Patent Office.

Nevertheless, great quantity of the purified C3dg must be obtained in order to evaluate the anti-inflammatory action of C3dg. If methods of obtaining C3dg, there is known only a method whereby a serum is treated at a temperature of 37°C or C3 is treated with a proteinase, such as Factor I, and the like, and the resulting C3dg then isolated with various HPLC (see, Japanese Patent Publication (Kokai) No. 2(1991)-91100). This method however, is not suitable for the aforementioned purposes, since the quantity of available C3dg is very limited.

To accomplish their purpose, the inventors carried out intensive research, and as a result, found a method of producing a great quantity of a protein comprising rat and human C3dg and having an inhibitory activity as high as that of the C3dg, to thereby complete this invention.

As described above, since the protein in accordance with the present invention specifically inhibits phospholipase $A_2$ at inflammatory sites, the protein is of interest as a preventive and remedial drug for particular diseases. Nevertheless, there is still a need for a more efficacious drug for allergies such as asthma, measles, or the like, which have recently become more wide spread.

It is known that allergic reactions can be classified into two types, i.e., the immediate type and the delayed type, and mast cells are a cause of an immediate allergic reaction. This is because mast cells have an IgE receptor having a high affinity in the surface of the cell membrane, release granule content such as a histamine, serotonin, and the like, by an antigenic sensitization (degranulation). Accordingly, a drug that inhibits an activation of the mast cells can be expected to have an improved efficacy with regard to allergies such as asthma and measles, etc., Also, the inventors have conducted research into an inhibitory protein for phospholipase $A_2$ originated from inflammatory sites and surprisingly, found that the inhibitory protein for phospholipase $A_2$ originated from inflammatory sites inhibits the degranulation of the mast cells, and thus another aspect of the present invention was completed.

Disclosure of the Invention

The present invention provides a plasmid comprising a DNA sequence encoding inhibitory protein for phospholipase $A_2$ originated from inflammatory sites, and a DNA sequence acting as a promoter to regulate an expression of the protein. Also, there is provided a recombinant microbial cell transformed with such a plasmid, and in addition to the cell, a method of producing the inhibitory protein for phosphlipase $A_2$ originated from influammatory sites, characterized in that the microbial cells are cultured in a nutrient medium to accumulate the protein, and the inhibitory protein is recovered from the cultures and inhibitory protein for phospholipase $A_2$ originated from inflammatory sites, produced by this method, is provided.

Further, in accordance with the present invention, a pharmaceutical composition for a suppression of an allergic reaction, comprising these inhibitory proteins, or any inhibitory protein for phospholipase $A_2$ originated from inflammatory sites and isolated and purified from body fluid or organs in mammals or degradated and prepared from a complement component C3 of mammals, is provided,

Brief Description of the Drawings

Figure 1 shows an outline of the construction of an expression plasmid for $\beta$-Galactosidase rat C3$\alpha$ fusion protein;
Figures 2 (a) and (b) show an outline of the construction of an expression plasmid for rat Met-C3dg;
Figures 3 (a) and (b) show an outline of the construction of an expression plasmid for human Met-C3dg;
Figures 4(a) and (b) show peaks of eluted proteins in fractions through gel filtration HPLC, and the results of SDS-PAG on the eluted fractions according to Example 5, respectively;
Figure 5 shows inhibitory activities of the fusion protein according to Example 7 for the phospholipase $A_2$ from rate inflammatory sites; and

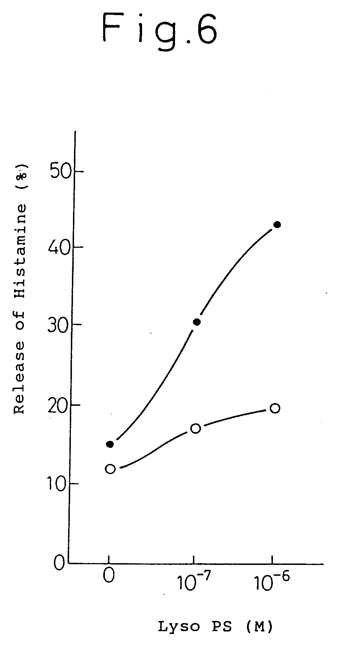

Figure 6 shows histamine releasing percentages (%) with the fusion protein according to Example 8, wherein is control, and o is the fusion protein.

Best Mode of Carrying Out the Invention

In accordance with the present invention, a "DNA sequence encoding an inhibitory protein for phospholipase $A_2$ originated from inflammatory sites" is any DNA sequence encoding a full amino acid sequence derived from C3dg, which is a degradation products of rat or human complement component C3, or DNA sequences encoding any amino acid sequences in which parts of amino acid in the sequence residues are mutually replaced, deleted from the sequence, or newly inserted into the sequence without an inactivation of the phospholipase $A_2$ inhibitory activity during the expression. For example, a specific full amino acid sequence of rat or human C3dg or a DNA sequence encoding the same is disclosed in WO91/01999, which is based on the inventor's application PCT/JP90/0096, and was published after filing date of Japanese Patent Application No. 2-205164, filed at the Japanese Patent Office on August 3, 1990 and claiming a priority from the present PCT application. Note, the above-mentioned reference is incorporated herein by reference thereto it.

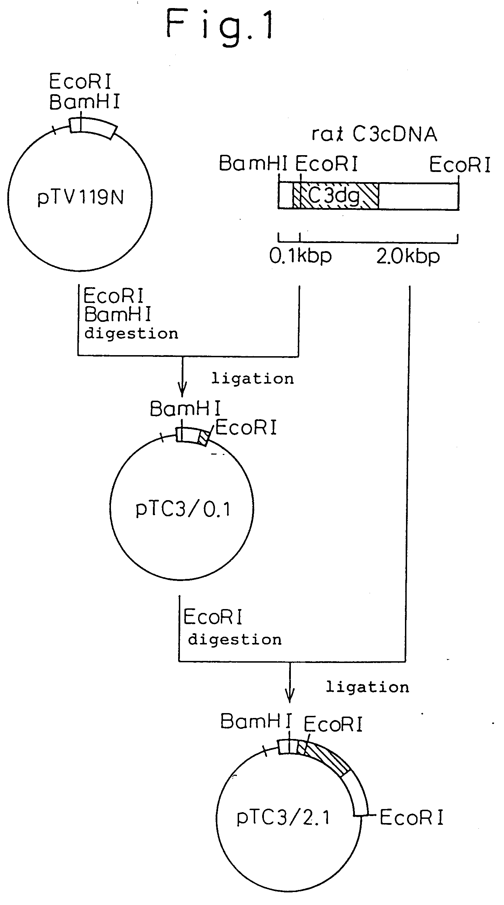

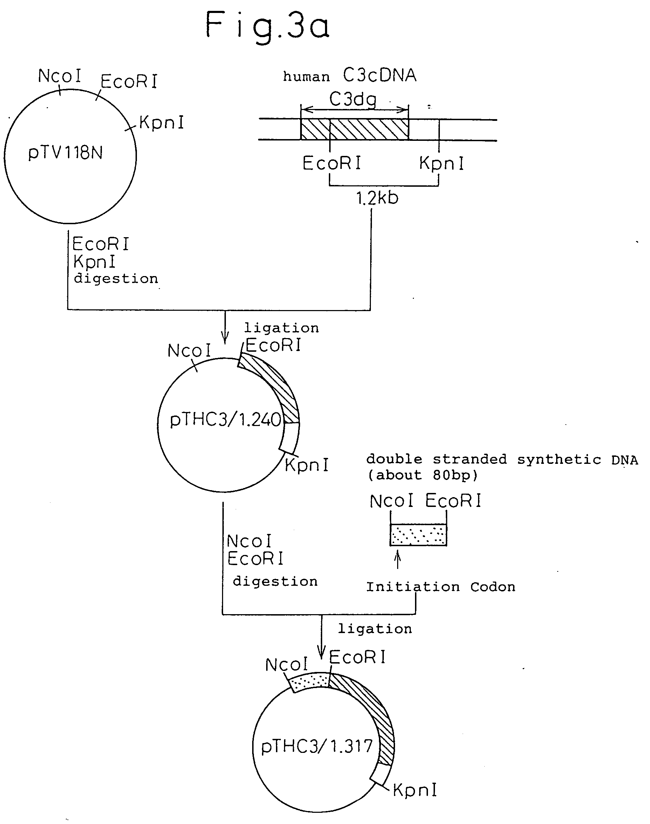

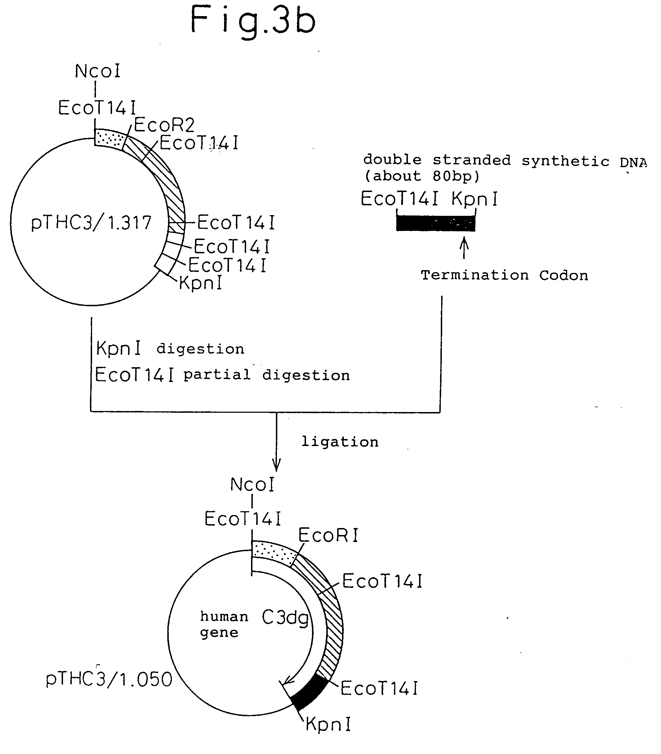

Such amino acid sequences are specifically described in the "Sequence Listing" sequence numbers 1 and 3 below. DNA sequences encoding such amino acid sequences are, for example, described in the "Sequence Listing," sequence numbers 2 and 4 below, included the sequences consisting of bases 1032 and 1047, respectively. In the construction of a plasmid of the present invention using such DNA sequences, the DNA sequences encoding a phospholipase $A_2$ inhibitory protein include any DNA sequence having an additional DNA sequence at upstream and/or downstream regions, as long as an expression protein of the DNA sequences has an activity equivalent to that of the former sequence. The additional DNA sequences include those generally associated with the desired sequence during a cloning of the same. Namely, for example, the DNA sequences in accordance with the present invention include any DNA sequence containing rat or human C3dg and prepared by the construction steps illustrates in Figure 1 and 3, in part thereof. For example, such DNA sequences include the following DNA sequences: a DNA sequence reconstructed by a ligation of a 2.1 kbp of a DNA fragment derived from rat liver cDNA λgtll libraries with a double stranded DNA, wherein the DNA fragment is reconstructed by using a 2.0 kbp of DNA fragment prepared by a digestion of the cDNA λgtll library using EcoRI, and a 0.1 kbp of DNA fragment prepared by a digestion of another 0.7 kbp of DNA fragment from the cDNA λgtll library using EcoRI, and the double stranded DNA is the 2.1 kbp of DNA fragment digested by EcoRI and FokI to form a 1.0 kbp of EcoRI - FokI fragment wherein, upstream of the EcoRI-FokI fragment, is added an initiation codon and a 5' terminal of about 60bp of rat C3dg gene, and then to the resulting DNA sequence is added a termination codon and a 3' terminal of about 20 bp of rat C3dg gene downstream thereof, as well as a reconstructed DNA sequence prepared by a ligation of an initiation codon and a 5' terminal of about 70 bp of human C3dg gene upstream of about 1.2 kb of EcoRI-KpnI fragment, which is a digested from human cDNA clone pHLC3.11 using EcoRI and KpnI followed by a ligation of a double-stranded DNA of an EcoT14I-KpnI fragment prepared by an addition of a 3' terminal of about 80 bp of a human C3dg gene and a termination codon downstream of about 0.97 kb of an NcoI - EcoT14I fragment prepared by a partial digestion of the double-stranded DNA of the NcoI-EcoRI fragment, using EcoT14I. Namely, a DNA sequence encoding the inhibitory protein for phospholipase $A_2$ (hereinafter referred to as "$PLA_2$") originated from inflammatory sites and utilized for the construction of the plasmid in accordance with the present invention, is conceptually described below:

(i) Init-$PLA_2$ • DNA-Term;

(ii) Init-Signal • DNA-$PLA_2$ • DNA-Term;

(iii)Init-a • DNA-b • DNA-$PLA_2$ • DNA-term; and

(iv) Init-Signal • DNA-a • DNA-b • DNA-$PLA_2$ • DNA-Term.

wherein Init represents an initiation codon, $PLA_2$ • DNA represents a DNA sequence encoding a $PLA_2$ inhibitory protein, Term represents a termination codon, Signal • DNA represents a DNA sequence encoding a signal peptide, a • DNA represents a DNA encoding an additional polypeptide to abundantly express the $PLA_2$ inhibitory protein in a host cell, and b • DNA represents a DNA sequence encoding a cleavable sequence of a peptide. Note, the above-mentioned terms are well known to those of ordinary skill in the art.

In the present invention, a DNA sequence having a promoter function includes a lactose-operon (lac) promoter and variant thereof (e.g., lac UV 5), a tryptophan (trp) promoter, a trp-lac hybrid promotor (e.g., tac, tacII, trc), a λ phage $P_L$ promoter and $P_R$ promoter, T7 promoter, tetracycline resistant gene (tet) promoter, trp-tet and lac-tet hybrid promoter, β-lactamase promoter, Escherichia coli outer membrane protein gene (lpp) promoter, synthetic promoter (e.g., DNA of bases 82 containing E. coli trp promoter and operator), and the like. Of these promoters, the lac promoter is particularly favored.

In the preparation of a plasmid in accordance with the present invention, the utilizable vectors include known or commercially available vectors per se, or vectors derived from the same, depending on the application. For example, it is convenient to prepare a plasmide wherein an expression vector is previously integrated with an element of the above-mentioned promoter sequences, Shine-Dalgarno (SD) sequences, terminator sequences, and the like. Further, other elements include the above-mentioned signal peptide coding DNA sequence; for example, if E. coli is employed as a host cell, these include alkaline phosphatase, OmpF, protein A, β-lactamase (penicillinase), lipoprotein, endotexin, and the like.

The expression plasmids include plasmids of the Col EI type such as pBR series, pUC series, etc., plasmids of the P15A type such as pACYC series, etc., plasmids of the F factor type such as mini F vector, etc., plasmids of the R factor type such as pSC101, etc., and plasmids derived from phage, such as pUC118 and 119, etc.. In particular, pTV118N, pTV119, and the like, which also comprise lac promoter, SD sequence, translational initiation codon, cloning site and terminator, are preferred.

Accordingly, in the plasmids in accordance with the present invention, preferably the DNA sequence encoding $PLA_2$ inhibitory protein is incorporated into a cloning site of the above-mentioned vector, and specifically, include ppTC3/2.1, pTRC3/1.035 and pTHC3/1.050. Such plasmids can be obtained by conventional methods from E. coli pTC3/2.1, J9TRC3/1.035 and J9THC3/1.050 strains; the E. coli JM109 strains being transformed by a conventional method using the above plasmids, i.e., ppTC3/2.1, pTRC3/1.035 and pTHC3/1.050, and deposited under deposit numbers FERM BP-3027 (transferred from FERM P-11431 deposited on April 26, 1990), FERM BP-3485 (deposited on July 17, 1991) and FERM BP-3486 (deposited on July 17, 1991), respectively, at the international depositary authority of the Fermentation Research Institute of Agency of Industrial Science and Technology, Japan.

The plasmids, including such plasmids can be prepared by an insertion of the DNA sequence encoding $PLA_2$ inhibitory protein to a cloning site of the above vector, for example, according to the construction procedures for plasmids known per se and shown in Figure 1, Figures 2(a) and (b), and Figures 3(a) and (b).

The recombinant microbial cells can be obtained by a transformation of a microbial cell with the plasmid described above. For this transformation, preferably a $CaCl_2$ method is used.

The host cells for the above-mentioned recombinant include, for example, microbial cells belonging to Eschericia, Bacillus, Saccharomyces, and the like. In particular, microbial cells belonging to Eschericia, and more particularly, Eschericia coil JM109, are preferred.

Thus, the recombinant microbial cells specifically enclose E. coli pTC3/2.1 (FERM BP-3027), E. coli J9TRC 3/1.035(FERM BP-3485), and E. coli J9THC 3/1.050 (FERM BP-3486), which are transformed using the preferred plasmids in accordance with the present invention.

The above-mentioned recombinant microbial cells are useful for the production of a protein having an inhibitory activity against phospholipase $A_2$ originated from inflammatory sites.

Namely, the present invention also provides a method of producing the inhibitory protein for phospholipase $A_2$ originated from inflammatory sites ($PLA_2$ inhibitory protein), and comprises culturing the recombinant microbial cells in a nutrient medium to produce the PLA$_2$ inhibitory protein, and recovering the inhibitory protein from the cultures. The culturing can be carried out under ordinary conditions for a culturing of E. coli, and further, a usual carbon source can be used as the nutrient medium, or a nitrogen source and other inorganic salts and trace amount of metals and vitamins can be used. According to the present method, the PLA$_2$ inhibitory protein is abundantly accumulated as the inclusion body in the microbial cells, or alternatively, the inhibitory protein is accumulated as extracellular protein in the cultured medium when the DNA sequences encoding a signal peptide are used.

When recovering the PLA$_2$ inhibitory protein from the cultured medium, the microbial cells are subjected to a lysis, such as a treatment with a cell wall lytic enzyme or sonication, to degrade the cell wall, or the microbial cells are removed from the cultured medium, followed by isolation and purification procedures, known per se, for proteins. More particularly, in the former case, the collected microbial cells are subjected to, for example, sonication, or the like to recover the inclusion bodies, the inclusion bodies are solubilized with urea, the treated solution is applied to a gel filtration column to remove the urea, and the resulting protein is subjected to pyridylethylation to protect SH groups.

The protein having protected SH groups is then fractionated with gel filtration HPLC to yield fractions containing mainly a recombinant protein derived from a C3α gene.

The fractions are further isolated and purified with a reverse phase HPLC, etc., based on an inhibitory activity against the rat phospholipase A$_2$ from inflammatory sites.

The above-mentioned inhibitory activity or inhibitory protein can be determined, or traced, as follows.

(A) Determination of Inhibitory Activity to Phospholipase A$_2$ from Inflammatory sites

Enzyme used was a phospholipase A$_2$ isolated and purified from the peritoneal cavity of caseinate-treated rats, and the substrate used was $^{14}$C-phosphatidylethanol amine isolated and purified from E. coli cultured together with $^{14}$C-acetic acid (about 2,000 dmp/nmol, 0.1 mM).

In the enzyme reaction, to a reaction mixture containing 50 μl of 0.5M Tris•HCl (pH 9.0), 25 μl of 40 mM CaCl$_2$, and 25 μl of substrate, a sample and water were added to a total volume of 240 μl, and finally, 10 μl of an enzyme solution (0.1 ng/μl) was added. The incubation was performed at 37°C for 10 minutes, and thereafter, to the reaction mixture was added Dole's reagent (1.5 ml), to stop the reaction, and the $^{14}$C-acetic acid generated was extracted using Dole's method, followed by a determination with a liquid scintillation counter.

(B) SDS-Polyacrylamide Gel Electrophoresis and Western-Blotting

To each of the samples prepared from the treated culture or the purification steps was added one tenth of dye solution (0.1% BPB, XC, 10% SDS) by volume, the solution was heated to 100°C for 5 min, and the reaction mixture then applied to 12.5% polyacrylamide gel with 1% SDS. Thereafter, an electrophoresis was performed at from 15 to 25 mA for 1.5 hours. When an analysis of the samples was carried out under a reductive condition, to each of the samples was added 2-mercaptoethanol (2ME). After the electrophoresis, the gels were subjected to CBB staining, for a detection of the protein.

Alternatively, after the electrophoresis as described above, the protein was transferred with an electro-blotting apparatus (MILLIPORE) from the gel to a nitrocellulose filter, and the gel was subjected to an enzyme-linked immunostaining method using anti-human C3d sheep serum (Dako), horseradish peroxidase conjugated anti-sheep IgG (Cappel) and 4-chloro-1-naphthol (Bio-Rad), to detect the C3d or C3dg band.

The phospholipase A$_2$ inhibitory protein from inflammatory sites thus obtained was, for example, comprised mainly by the amino acid sequences represented in the "Sequence Listing" sequence numbers 1 or 3. As described below, it was confirmed that the proteins have a very specific inhibitory activity against the phospholipase A$_2$ originated from inflammatory sites, and further, have suppressive effect on allergic reactions. It was also confirmed that proteins known to specifically inhibit phospholipase A$_2$ from inflam-matory sites, such as the protein originated from rat peritoneal cavity as disclosed in Japanese Unexamined Patent Publication (Kokai) No. 63(1988)-246397, the protein prepared by using an enzyme from a human complement component C3 as disclosed in Japanese Unexamined Patent Publication (Kokai) No. 2 (1990)-91100, and the proteins purified from rat or human serum disclosed in International Publication No. WO91/1999, have the above-mentioned suppressive effect on allergic reactions. Surprisingly, it was found that this effect is due to fact that such inhibitory proteins inhibit a degranulation of mast cells.

Accordingly, another aspect of the present invention is directed to a pharmaceutical composition for a suppression of allergic reaction containing the phospholipase A$_2$ inhibitory protein as an active ingredient. The inhibitory protein includes a protein obtainable through genetic engineering procedures according to the invention, a protein isolated and purified from mammals and a protein prepared from the complement component C3.

The term "allerigic reaction" means a reaction occurring when an antigenic sensitized organism is again subjected to the same antigenic sensitization, and in particular, includes an immediate allergy such as anaphylaxis and Arthus reaction induced in mast cells. As mammalian, in particular human, diseases discussed herein, asthma, measles, and the like, are particularly mentioned.

In the present invention, an oral drug such as a soft capsule, a hard capsule, a tablet, or a syrup, an injection, or a surgical drug, all of which may be prepared by using the PLA$_2$ inhibitory proteins as an active ingredient and an appropriate excipient in a proper known manner may be utilized.

The dosage of the active ingredient is usually about 1 to 500 mg/day/person and the number of doses is usually 1 to 3 times/day, and thus a preparation fulfilling such conditions is preferably prepared. Further, the active ingredient does not show an acute toxicity to mammals in the range of the above-mentioned dose. The depressant of the present invention also may be used together with existing drugs.

The efficacy of the PLA$_2$ inhibitory proteins with respect to a pharmaceutical composition for the suppression of allergic reactions is illustrated by the following tests.

(C) Determination of Inhibitory Activity (Suppression of
Histamine Release) upon IgE-Antigen-Lyso-PS Dependent Activation of Rat CTMC

(1) Preparation of Rat Connective Tissue Type Mast Cell (CTMC)

The peritoneal cavities of male Whistar rats (having a weight of not less than 500 g) are each injected with 50 ml of a Hank's solution containing 10 $\mu$/ml of heparin, and massaged more than 100 times. The Hank's solution is collected by opening the pertoneal cavity. The cells are collected by centrifugation at 100 x g for 5 minutes, and then the Hank's solution is collected. After being suspended in 1 mℓ of Hank's solution, the suspension is gently layered on 5 mℓ of a Hank's solution in which 40% bovine serum albumin (BSA) had been dissolved, and centrifuged at 450 x g for 20 minutes at 4°C. The cells residing in the lowermost layer are collected and washed with a Hank's solution, to obtain peritoneal cavity mast cell (purity: more than 90%). About 10$^6$ cells are obtained per rat.

(2) Method of Activation of Rat CTMC

The resulting peritoneal cavity mast cells are resuspended in a Hank's solution containing 0.1% gelatine (5 x 10$^6$/mℓ), and 1 $\mu$g/mℓ of anti-DNP IgE-antibody (Miles) is added thereto to be subjected to passive sensitization. The cells are washed, and re-suspended in the Hank's solution containing 0.1% gelatine to a cell concentration of 1 to 2 x 10$^6$/mℓ, an adequate amount of antigen, DNP-Ascharis [a method according to Eisen et. al (I. Am. Chem. Soc., 75. 4583 (1953))], and 10$^{-6}$ M lysophosphatidylserine (Avanti) are added thereto, and the mixture is incubated at 37°C for 10 minutes to activate the cells. The reaction is stopped by adding EDTA, to be 2 mM.

(3) Determination of Histamine Released from Activated Rat Mast Cell

(a) Preparation of Histamine N-Methyltransferase

An SD rat's kidney (200 g) is homogenized with 0.25 M sucrose solution in a volume 9 times the wet weight of the kidney, and centrifuged at 40,000 x g for 1 hour. To the supernatant is added ammonium sulfate, and 45-70% ammonium sulfate precipitated fraction is recovered, and then is dissolved in 0.1 M phosphate buffer (pH 7.4) and dialyzed against a 0.01 M phosphate buffer (pH 7.4). This standard is used as the HMT.

(b) Determination of Histamine

A sample containing histamine (19-50 $\mu$ℓ) is placed in a test tube, an adequate amount of HMT and 1$\mu$ℓ of $^3$H (tritiated) S-adenosylmethionine (New England Nuclear) are added thereto, and a 0.1 M phosphate buffer (pH 7.9) is added to adjust the total volume to 200 $\mu$ℓ. After incubation at 37°C for 90 minutes, the reaction is stopped by the addition of 100 $\mu$ℓ of 2.5 N NaOH. Subsequently, 2 mℓ of chloroform is further added to extract the $^3$H-methyl histamine into the chloroform layer. The water layer and chloroform layer are separated by centrifugation at room temperature for 5 minutes, and the water layer is then removed. To the remaining chloroform layer is added 200 $\mu\ell$ of 2.5 N NaOH, to carry out the extraction again, thereby washing the chloroform layer. A certain amount of the resulting chloroform layer is dispensed in a vial bottle, and dried to form a solid, and there after, a toluene type scintillator is added to count the radioactivity.

Example

The following examples are illustrative of the invention, and in no way limit same.

Example 1 (referential example): Cloning of Inhibtory Protein for Phospholipase A$_2$ originated from Inflammatory Sites

(A) A plasmids pFC4/5.4 containing a mouse C3 cDNA fragment (J.B.C. 260, 10936, 1986) were digested with Hind III, and the resulting digested material was applied to a low melting point agarose gel electrophoresis to separate 2.2 kbp fragments. Further, 1.8 kbp fragments were obtained from Stu I digested material using the same procedure.

(B) Screening of cDNA

Rat liver cDNA λgt II libraries (Clontech) were used as the material for screening, and the screening procedures were carried out according to the protocol of Clontech.

Namely, λgt II phage infected E. coli Y 1090 strains were cultured at 37°C for 5 hours to yield about 5 x 10$^4$ transformants, the resulting transformants were subjected to replication on a Nylon filter membrane, the filter was dipped in 1.5M NaOH-1.5M NaCl to be denatured and then neutralized with Tris•HCl buffer (pH 7.2) containing 1.5M NaCl - 1mM EDTA. The filter was then dried by air, and exposed to ultraviolet rays from a trans-illuminator, to immobilize DNA on the filter.

As a probe for the screening, $^{32}$P labelled mouse C3 cDNA fragments, as described (A), were used.

The transformations on the test filter were hybridized with the probe at 65°C, for the screening, and then detected by autoradiography. As a result, seven positive clones were found among the about 5 x 10$^4$ transformants, and these positive clones were designated as λrC3/11-17, respectively.

The above clones were digested with EcoRI, subjected to agarose gel electrophoresis, and then the chain length of insert genes was determined by Southern hybridization. As a result, it was found that λrC3/11 of the above-mentioned clones had the longest insert gene fragment, in which the full length is 2.7 kbp, and were digested with EcoRI to yield 0.7 kbp and 2.0 kbp fragments.

(C) Sequence Determination for Rat C3 cDNA

After λrC3/11 phage DNA's were isolated from the clone, the resulting DNA's were digested with EcoRI to yield DNA 0.7 kbp and 2.0 kbp fragments. These fragments were inserted into the EcoRI cleavage sites of a cloning vector pUC19 and phage M13MP 19RE DNA (Takara Shuzo) for sequencing. The 0.1 kbp and 0.6 kbp fragments from the 0.7 kbp fragment were inserted in a sequencing vector pUC118 double digested with EcoRI and BamHI, and were subjected to sequencing from both terminals.

The sequence determination was made in accordance with the 7-DEAZA method (Mizusawa, et al., Nucreic Acid Res. 14, 1319, 1986). In the sequencing of a 2kbp fragment, the method of Hood et al. (Hood, et al, Anal. Biochem. 154, 353, 1986), in which primers corresponding to parts of clarified sequences are synthesized and a sequence determination conducted, was used.

Following the above procedures, a sequence determination as described in International Publication No. WO 91/01999 was carried out, and as a result, an full nucleotide sequence obtained and its deduced amino acid sequence of rat inhibitory protein for phospholipase A$_2$ originated from inflammatory sites are shown as "Sequence Listing," sequence numbers 3 and 1, respectively.

Also, an full nucleotide sequence obtained and its deduced amino acid sequence of human inhibitory protein for phospholipase A$_2$ originated from inflammatory sites, according to a sequencing in a similar manner as the above-mentioned procedures, are shown as "Sequence Listing," sequence numbers 4 and 2, respectively.

Example 2: Construction of C3α Expression Plasmid The procedure of the construction was based on Fig. 1.

An E. coli expression vector pTV119N (Takara Shuzo) was digested with BamHI and EcoRI. A rat C3 cDNA clone pTC3/11 was digested with BamHI and EcoRI, the fragment purified by agarose gel electrophoresis was separated, and a 0.1 kb BamHI-EcoRI fragment and 2.0 kb EcoRI-EcoRI fragment were recovered.

The ligation reaction was carried out between the digested vector and 0.1 kb fragment, and E. coli, JM109 was transformed, and there after, was cultured on an ampicillin plate to yield a transformant. The plasmid DNA was extracted and purified from the transformant, and the plasmid was designated as "ppTC3/0.1".

The plasmid "ppTC3/0.1" was digested with EcoRI, and then subjected to a ligation reaction together with the 2.0 kb fragment. After the plasmid DNA was extracted and purified from the transformant in a similar manner, the nucleotide sequence was detected at the BamHI site by the dideoxy method. From the nucleotide sequence, the plasmid that had been correctly inserted was selected, and was designated as "ppTC3/2.1".

In the above-procedure, the vector DNA and restriction enzymes were obtained from Takara Shuzo. In the recovery of the DNA from the agarose gel, and in the purification of the plasmid from the transformant, GENECLEAN available from BIO 101 Corp was used. The ligation reaction was carried out using a ligation kit produced by Takara Shuzo. The detection of the nucleotide sequence was carried out by using an M13 Primer RV-N and 7-DEAZA Sequencing Kit, both from Takara Shuzo Co., Ltd.

Since the vector, pTV119N, used herein possesses a lac promoter, the expression of the desired protein is induced by the addition of IPTG to the medium. The protein, which is expressed by ppTC3/2.1, is a fusion protein having 16 residues of N-terminal portions of E. coli $\beta$-galactosidase on the N-terminal side and 714 residues residing downstream of the rat 3$\alpha$-chain on the C-terminal side, and having a molecular weight of about 80 kDa.

The fusion protein is produced by culturing the recombinant E. coli pTC3/2.1 strain (FERM BP-3027) so that E. coli JM109 is transformed with the ppTC3/2.1 according to the potassium chloride method.

Example 3: Construction of Rat Met-C3dg Expression Plasmid

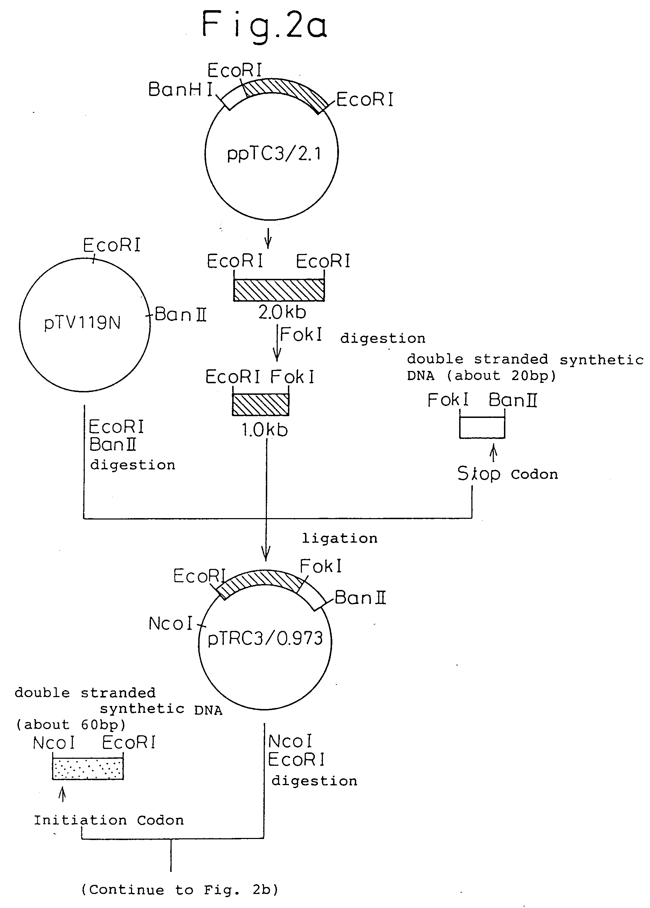

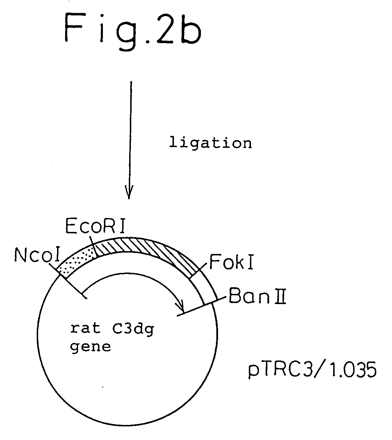

The construction procedure followed that shown in Figures 2(a) and (b).

The plasmid ppTC3/2.1 was digested with EcoRI to yield 2.0 kb of an EcoRI-EcoRI fragment, and the obtained fragment was further digested with FokI to yield 1.0 kb of an EcoRI-FokI fragment. A DNA was synthesized such that a protein synthetic termination codon TGA was added to 14bp from the 3' terminal of a rat C3dg gene, based on a DNA sequence of the rat C3dg, and then subjected to annealing to form a double-stranded synthetic DNA fragment of FokI-BanII. An expression vector pTV119N (Takara shuzo) was digested with EcoRI and BanII, and the fragment thus obtained was subjected to a ligation reaction together with 1.0 kb of an EcoRI-FoKI fragment and the synthetic DNA fragment of FoKI-BanII, and E. Coli JM109 strains were transformed with the fragments and then cultured on an LB agar medium containing ampcillin, to yield transformants. A plasmid DNA extracted from the transformants was purified, and designated as "pTRC3/0.973".

The plasmid pTRC3/0.973 was digested with NcoI and EcoRI. A DNA was synthesized such that, upstream of a 5' terminal 596p of rat C3dg gene was ligated a protein synthetic initiation codon ATG, and then subjected to annealing, to yield two double-stranded NcoI - EcoRI fragments. The ligation reaction was carried out between the synthesized DNA fragment and digested pTRC3/0.973 and E. coli JM 109 was transformed, and then after, was cultured on an LB agar medium containing ampcillin, to yield transformants. The plasmid DNA was extracted and purified from the transformants, the nucleotide sequence was detected upstream at 26 bp of the NcoI cleaved site and downstream at 41 bp of the BanII cleaved site by the dideoxy method, and a plasmid having inserted therein 1032 bp of rat C3dg cDNA between the protein synthetic initiation codon and termination codon, was designated as plasmid "pTRC3/1.035".

pTRC3/1.035 was used for a transformation of the JM109 to yield J9TRC3/1.035 (FERM BP-3485).

Since the vector pTV119N used herein possesses a lac promoter, the transformants cultured in a medium containing IPTG (iso-propyl-thiogalactoside) can be induced to express the desired protein. The expression protein from pTRC3/1.035 has methionine at the N terminal of rat C3dg 344 residue, and a molecular weight of about 39 kDa.

Example 4: Construction of Human Met-C3dg Expression Plasmid

The construction procedure was based on Figures 3(a) and (b).

An E. coli expression vector pTV118N (Takara Shuzo) was digested with EcoRI and KpnI, a human C3 cDNA clone pHLC3.11 obtained from ATCC (American Type Culture Collection, U.S.A.) was digested with EcoRI and KpnI, and there after, 1.2 kb of an EcoRI - KpnI fragment was separated and recovered by agarose gel electrophoresis.

The ligation reaction was carried out between the digested vector and the 1.2kb fragment, and E. coli JM109 was transformed, following by culturing on an LB agar medium to yield a transformant. The plasmid DNA was extracted and purified from the transformant, and was designated as "pTHC3/1.240.

The plasmid pTHC3/1.240 was digested with NcoI and EcoRI, and then subjected to agarose gel electrophoresis to yield 4.4 kb of an NcoI - EcoRI fragment. 5' terminal about 70 bp of human C3dg cDNA downstream of a protein synthetic initiation codon ATG was synthesized according to a known human C3dg cDNA nucleotide sequence, and subjected to annealing to yield a double-stranded NcoI - EcoRI fragment.

The ligation reaction was carried out between the 4.4 kb fragment from pTHC3/1.240 and the synthesized DNA fragment, and E. coli JM109 was transformed, followed by culturing on an LB agar medium containing ampicillin to yield a transformant. The plasmid was extracted and purified from the transformant, and was designated as "pTHC3/1.317".

The plasmid pTHC3/1.317 was completely digested with KpnI, and further, partially digested with EcoT14I, where after 4.0 kb of KpnI - EcoT14I fragment was separated and recovered by agarose gel electrophoresis. Further, a DNA ligated protein synthetic termination codon TAG to a 3' terminal of about 80 bp of human C3dg cDNA was synthesized according to a human C3dg cDNA nucleotide sequence, and was subjected to annealing to yield a double-stranded EcoT14I - KpnI fragment.

The ligation reaction was carried out between the 4.0 kb fragment from pTHC3/1.317 and the synthesized fragment, and E. coli was transformed, whereafter it was cultured on an LB agar medium containing ampcillin to yield a transformant. The plasmid DNA was extracted and purified from the transformant, and then from 26 bases upstream of NcoI cleavaged site and 43 bases downstream of a KpnI cleaved site, the nucleotide sequence was determined by the dideoxy method. As a result, the plasmid having inserted therein 1047 bp of human C3dg cDNA, between the protein synthesis initiation codon and termination codon, was designated as "pTHC3/1.050".

Using thus obtained pTHC3/1.050, JM109 was transformed by the ordinary calcium chloride method to yield the recombinant E. coli J9THC3/1.050 (FERM BP-3486).

Since the vector, pTV119N, used herein possesses a lac promoter, the expression of the desired protein is induced by the addition of IPTG to the medium. The protein expressed by pTHC3/1.050 has methionine at the N terminal of human C3dg 349 residue, and a molecular weight of about 39 kDa.

Example 5: Expression of Inhibitory Protein for $PLA_2$ from purified Inflammatory Sites in E. coli and Purification of Expressed Protein

After pTC3/2.1 (FERM BP-3027), J9TRC3/1.035 (FERM BP-3485) or J9THC3/1.050 (FERM BP-3486) had been cultured in 10 mℓ of 2xTY medium containing 50 μg/mℓ of sodium ampicillin at 37°C overnight, the whole amount was transferred to a total volume 1 ℓ of the same medium, and the culture was carried out at 37°C. After about 5 hours, IPTG was added to a final concentration of 100 μM, and the culture was continued for another 3 hours.

The cells of cultures were independently collected, and 2-ME and SDS were each added thereto, the total proteins were subjected to SDS-PAGE for a separation, and the separated proteins then transferred by an electro-blotting apparatus from Bio-Rad Co. LTD. to a nitrocellulose filter. After the transfer, the filter membranes were processed by blocking with 20 mM. PBS (pH 7.5) containing 3% BSA, and then the proteins were reacted with each dilute solution in which, in the case of pTC3/2.1, were goat anti-rat C3 serum (CAPPEL), and in the case of J9TRC3/1.035 and J9THC3/1.050, were rabbit anti-human C3d serum (DAKO) diluted with PBS containing 1% BSA by 100 times, respectively, at 4°C for 4 hours. After they were washed by PBS, the proteins were independently reacted with each dilute solution which, in the pTC3/2.1 case, was swine anti-goat IgG antibody-HRPO complex (TAGO), in the J9TRC3/1.035 and J9THC3/1.050 cases was sheep anti-rabbit IgG antibody-HRPO complex (Bio-Rad), diluted with PBS containing 1% BSA by 500 times, respectively, at 4°C for 2 hours. After they were washed by PBS, the membranes were stained with a Konica Immunostain HPR Kit, and as a result, it was confirmed that, with respect to pTC3/2.1 a rat C3α fusion protein having a molecular weight of about 80 kDa, with respect to J9THC3/1.050, a Met-human C3dg having a molecular weight of about 39 kDa, and with respect to J9TRC3/1.035, a Met-rat C3dg having a molecular weight about 39 kDa, were expressed, respectively.

With respect to pTC3/2.1, the remaining culture was further subjected to centrifugation at 3,000 G for 5 minutes to collect the cells, and suspended in 40 mℓ of 50 mM Tris.HCℓ (pH 7.5) buffer, after which a sonication (Kubota INSONATRON 201M) was conducted at the maximum output for 5 minutes to destory the cells. The inclusion bodies were collected by centrifugation at 3,000 G for 10 minutes, and 10 mℓ of 8M urea-100 mM Tris HCl buffer (pH 8.0) was added thereto, after which the mixture was left standing at room temperature for 30 minutes to solubilize the inclusion proteins. After insolubles were removed by centrifugation at 10,000 g for 10 minutes, the solution was applied to a gel filtration column (PD 10 column,

10

Pharmacia) in order to remove urea, and eluted with 10 mM ammonium acetate, lyophilization by freeze-drying.

About 30 mg of the lyophilized sample was dissolved in 2 m$\ell$ of 0.2 M ethylmorpholine acetate (pH 8.0) containing 1% SDS, 2 $\mu\ell$ of 2-mercaptoethanol (2ME) was added thereto, and the mixture was left to stand at room temperature for 20 minutes. After 2 $\mu\ell$ of 2ME had been newly added, and the mixture was left to stand at room temperature for another 30 minutes, 3 $\mu\ell$ of 4-vinyl pyridine was added to cause a reaction for 1 hour.

After 3 $\mu\ell$ of 4-vinyl pyridine was been further added, to bring about a reaction at room temperature for another 1 hour, the reaction mixture was immediately applied to a gel filtration HPLC (TSKgel G3000SW). The elution was carried out with 50 mM Tris•HC$\ell$-150 mM NaC$\ell$ (pH 7.5) at 0.5 m$\ell$/min.

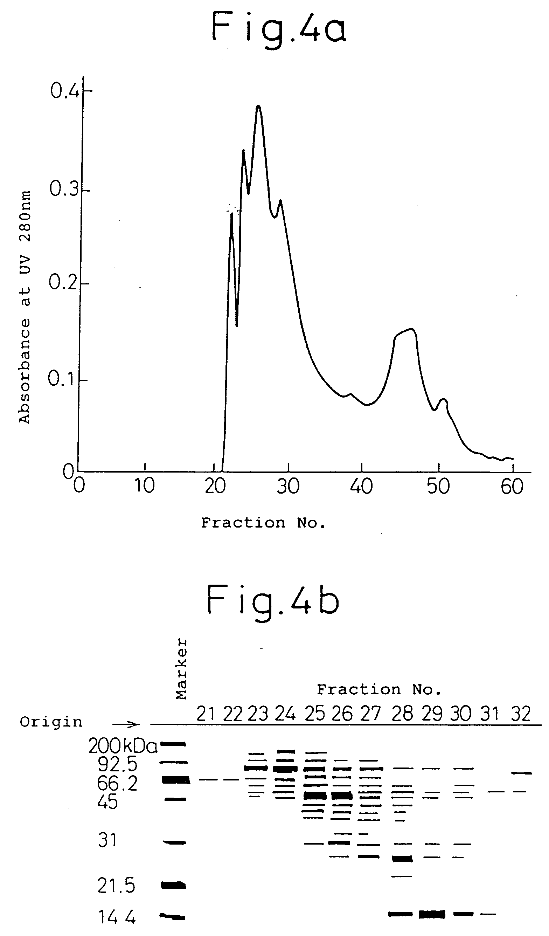

When the gel filtration fractions were analyzed by SDS-PAGE, the 80 kDa fusion protein was proven to be eluted around the 24th fraction (Figures 4(a) and (b). This fraction was further purified with reverse phase HPLC (Vydac, Protein C4) to give the final sample.

The amount of protein purified from 1 $\ell$ of the E. coli. culture was about 600 $\mu$g.

In the above-described procedures, the HPLC was carried out using a Spectra-Physics pump system SP 8700, applied spectroscopic UV detector UVILOG-511 A, LKB fraction collector 2112, and Reodine sample injector 7125. The SDS-PAGE was carried out using a 5-20% gradient gel (ATTO Pagel), and stained with CBB (Sigma).

Example 6: Identification of Purified Protein

(1) Identification by immunoblott Method

After the gel filtration HPLC fraction had been separated with SDS-PAGE, the gel was shaken in a 25 mM Tris-HCl-192 mM glycine buffer containing 20% methanol for 30 minutes, and the transfer onto a nitrocellulose membrane was carried out by using an electroblotting apparatus (Immuno Blot, Bio-Rad) 0.5 A, 30 minutes).

The transferred membrane was processed by blocking with 20 mM PBS (pH 7.5) containing 3% BSA while shaking for 1 hour, and then reacted with goat anti-rat C3 antibody (CAPPEL) which had been diluted with 20 mM PBS by 100 times at 4°C for 4 hours.

After washing three times with PBS containing 1% Tween-20, it was then reacted with goat anti-rat IgG antibody-HRPO complex (TAGO) which had been diluted with PBS containing 1% BSA by 500 times, at 4°C for 2 hours, and after washing three times with PBS containing 1% Tween-20, it was stained with the Konica Immunostain HPR kit.

As a result, it was confirmed that the 80 kDa protein band eluting around #24 had been reacted with the anti-rat C3 antibody, and thus was the desired fusion protein.

(2) Detection of N-Terminal Amino Acid Sequence

After the final sample purified with reverse phase HPLC was dried by using a vacuum centrifugal concentrator, it was dissolved in 80% acetonitrile-0.1% TFA, and applied to a gas phase protein sequenced (Applied Biosystems, Model 477A) to determine the N-terminal amino acid sequence.

As a result, it was proven that the N-terminal amino acid sequence of 80 kDa fusion protein was determined to be the following amino acid sequence;

$NH_2$-Ala-Met-Ile-Thr-Pro-X-Ser-

This sequence agreed with the sequence expected, although only the first Met is deleted from the E. coli $\beta$-galactosidase. It could be considered that the N-terminal Met had been deleted in some mechanism after the synthesis of the desired 80 kDa fusion protein.

Example 7: Phospholipase A$_2$ Inhibitory Activities of C3$\alpha$-Chain Fusion Protein

The phospholipase A$_2$ inhibitory activities of the gel filtration HPLC fraction #24 was determined.

The activity determination system was prepared by adding distilled water to 100 mM Tris•HCl (pH 9.0), 4 mM Calcium chloride, 0.1 mM [$^{14}$C]-phosphatidylethanol amine (2,000 dpm/nmol), and 10-100 $\mu\ell$ of sample to attain a total amount of 240 $\mu\ell$, mixing them, and finally adding 10 $\mu\ell$ of 0.1 ng/$\mu\ell$ phospholipase A$_2$ thereto. In this case, phospholipase A$_2$ secreted from rat platelet was used (the same as the inflammatory site-originated type). The phosphatidylethanolamine was purified from E. coli, which had been cultured in a medium containing [$^{14}$C]-acetic acid. The reaction was carried out at 37°C for 10 minutes, with shaking. The termination of the reaction and the extraction of the fatty acid were carried out according to the Dole's method, and the extracted [$^{14}$C]-fatty acid was measured with a scintillation counter.

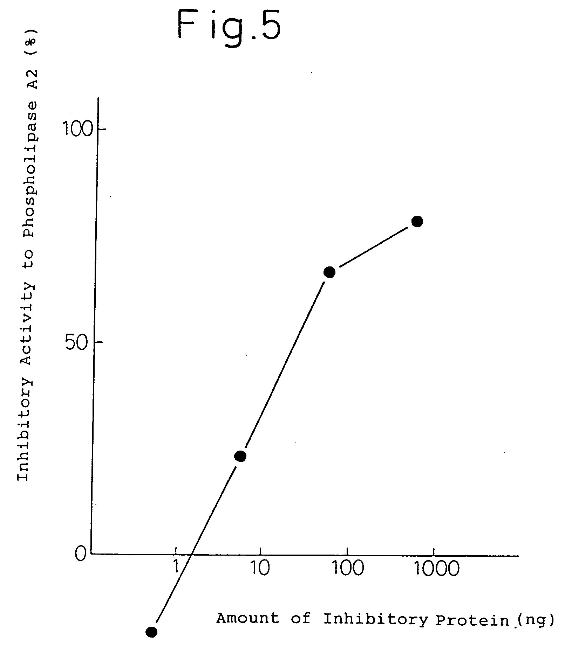

The results are shown in Figure 5. The present fraction inhibited the phospholipase $A_2$ from platelet in a dose dependent manner. The amount of the protein required for inhibiting 1 ng of the phospholipase $A_2$ activity 50% was about 50 ng. The specific activity per molecular weight of the inhibitory protein was about 1/5 times that of the rat C3dg purified from the serum (see, W091/01999)

Additionally, when the protein was evaluated for inhibitory activities against various phospholipase $A_2$ , although it had a highly inhibitory activity against phospholipase $A_2$ from rat inflammatory sites, phospholipase $A_2$ from human inflammatory sites, and phospholipase $A_2$ from Crotalus adamanteum venom similar to that of rat C3dg purified from rat platelet, the protein had no inhibitory activity against phospholipase $A_2$ from swine spleen and phospholipase $A_2$ from Naja naja venom.

Example 8: Effects of the Rat C3α Fusion Protein upon IgE-Antigen-Lysophosphatidylserine (ps)-Dependent-Activation of Rat CTMC

The activation of mast cells described under the above-described (C) (2) was repeated, but the rat C3α-chain fusion protein obtained in Example 5 was added simultaneously with the antigen and lyso-PS. That in which no inhibitory protein had been added was measured as a control. As a result, the release of histamine was inhibited as shown in Fig. 5. In combination with the results of Example 7, it was clarified that the present fusion protein inhibited type II PLA$_2$ , whereby the release of histamine was inhibited. In the Figure, the mark o represents control, and o is the present protein.

Although the concentration of tranilast, which is a depressant for release of histamine, required for exhibiting preferential release depressing activity is $10^{-4}$ M, in the case of the rat C3α-chain fusion protein, the concentration is 2.5 x $10^{-7}$ M, showing that the present protein clearly possesses a very strong activity for depressing inflammatory reactions.

Industrial Applicability

The present plasmid and recombinant microbial cell, and the method of producing the phospholipase $A_2$ inhibitory protein using the same, may be useful, for example, for a production of a remedy for mammals, especially human, allergies, since the protein has a suppressive action against allergic reactions thereof.

Reference to Deposited Microorganisms

International Depository Authority: Fermentation Research Institute, Agency of Industrial Science and Technology
Address: 1-3 Higashi I chome, Tsukuba-shi, Ibaraki-ken, 305 Japan
Deposition Number and Date:
1. FERM BP-3027: April 26, 1990 (transferred from FERM P-8585 deposited on April 26, 1990)
2. FERM BP-3485: July 17, 1991
3. FERM BP-3486: July 17, 1991

Sequence Listing

NUMBER OF SEQUENCE:  1

LENGTH OF SEQUENCE:  344

TYPE OF SEQUENCE:  amino acid

TOPOLOGY:  linear

MOLECULE TYPE:  protein

FRAGMENT TYPE:  middle fragment

SEQUENCE:

```
Glu Asp Val Pro Ala Ala Asp Leu Ser Asp Gln Val Pro Asp Thr
 1               5                   10                  15

Asp Ser Glu Thr Arg Ile Leu Leu Gln Gly Thr Pro Val Ala Gln
                20                  25                  30

Met Ala Glu Asp Ala Val Asp Gly Glu Arg Leu Lys His Leu Ile
                35                  40                  45

Val Thr Pro Ser Gly Cys Gly Glu Gln Asn Met Ile Gly Met Thr
                50                  55                  60

Pro Thr Val Ile Ala Val His Tyr Leu Asp Gln Thr Glu Gln Trp
                65                  70                  75

Glu Lys Phe Gly Leu Glu Lys Arg Gln Glu Ala Leu Glu Leu Ile
                80                  85                  90

Lys Lys Gly Tyr Thr Gln Gln Leu Ala Phe Lys Gln Pro Ser Ser
                95                  100                 105

Ala Tyr Ala Ala Phe Asn Asn Arg Pro Pro Ser Thr Trp Leu Thr
                110                 115                 120

Ala Tyr Val Val Lys Val Phe Ser Leu Als Ala Asn Leu Ile Ala
                125                 130                 135

Ile Asp Ser Gln Val Leu Cys Gly Ala Val Lys Trp Leu Ile Leu
                140                 145                 150

Glu Lys Gln Lys Pro Asp Gly Val Phe Gln Glu Asp Gly Pro Val
                155                 160                 165

Ile His Gln Glu Met Ile Gly Gly Phe Arg Asn Thr Lys Glu Ala
                170                 175                 180

Asp Val Ser Leu Thr Ala Phe Val Leu Ile Ala Leu Gln Glu Ala
                185                 190                 195

Arg Asp Ile Cys Glu Gly Gln Val Asn Ser Leu Pro Gly Ser Ile
                200                 205                 210

Asn Lys Ala Gly Glu Thr Leu Glu Ala Srr Tyr Leu Asn Leu Gln
                215                 220                 225

Arg Pro Tyr Thr Val Ala Ile Ala Gly Tyr Ala Leu Ala Leu Met
                230                 235                 240

Asn Lys Leu Glu Glu Pro Tyr Leu Thr Lys Phe Leu Asn Thr Ala
                245                 250                 255

Lys Asp Arg Asn Arg Trp Glu Glu Pro Gly Gln Gln Leu Tyr Asn
                260                 265                 270
```

Val Glu Ala Thr Ser Tyr Ala Leu Leu Ala Leu Leu Leu Leu Lys
                   275           280               285

Asp Phe Asp Ser Val Pro Pro Val Val Arg Trp Leu Asn Glu Gln
                   290           295               300

Arg Tyr Tyr Gly Gly Gly Tyr Gly Ser Thr Gln Ala Thr Phe Met
                   305           310               315

Val Phe Gln Ala Leu Ala Gln Tyr Gln Thr Asp Val Pro Asp His
                   320           325               330

Lys Asp Leu Asn Met Asp Val Ser Leu His Leu Pro Ser Arg
                   335           340

NUMBER OF SEQUENCE:   2

LENGTH OF SEQUENCE:   1032

TYPE OF SEQUENCE:  nucleic acid

STRAND TYPE: double

TOPOLOGY:  linear

MOLECULE TYPE:  cDNA to mRNA

FRAGMENT TYPE: middle fragment

IMMEDIATE SOURCE

      •Library Name:  rat liver cDNAλgtll

      •Clone Name: ppTC3/2.1

SEQUENCE:

GAGGATGTAC CTGCAGCAGA CCTCAGTGAC CAAGTGCCAG ACACAGATTC TGAGACCAGA
60

ATTCTCCTGC AAGGGACCCC GGTGGCTCAG ATGGCCGAGG ACGCTGTGGA CGGGGAGCGG
120

CTGAAACACC TGATCGTGAC CCCCTCTGGC TGTGGGGAGC AGAACATGAT TGGCATGACA
180

CCCACGGTCA TTGCAGTACA CTATCTGGAT CAGACCGAAC AGTGGGAGAA ATTCGGCCTA
240

GAGAAGAGGC AAGAAGCTCT GGAGCTCATC AAGAAAGGGT ACACCCAGCA GCTGGCTTTC
300

AAACAGCCCA GCTCTGCCTA TGCTGCCTTC AACAACCGGC CTCCCAGCAC CTGGCTGACA
360

GCCTATGTGG TCAAGGTCTT CTCTCTGGCT GCCAACCTCA TCGCCATCGA CTCTCAGGTC
420

CTGTGTGGGG CTGTCAAATG GCTGATTCTG GAGAAACAGA AGCCAGATGG TGTCTTTCAC
480

GAGGACGGAC CAGTGATTCA CCAAGAAATG ATTGGTGGCT TCCGGAACAC CAAGGAGGCA
540

GATGTGTCGC TTACAGCCTT TGTCCTCATC GCACTGCAGG AAGCCAGAGA TATCTGTGAG
600

GGGCAGGTCA ACAGCCTTCC CGGGAGCATC AACAAGGCAG GGGAGTATCT TGAAGCCAGT
660

TACCTGAACC TGCAGAGACC ATACACAGTA GCCATTGCTG GGTATGCCCT GGCCCTGATG
720

AACAAACTGG AGGAACCTTA CCTCACCAAG TTTCTGAACA CAGCCAAAGA TCGGAACCGC
780

TGGGAGGAGC CTGGCCAGCA GCTCTACAAT GTGGAGGCCA CCTCCTACGC CCTCCTGGCC
840

CTGCTGCTGC TGAAAGACTT TGACTCTGTG CCTCCTGTGG TGCGCTGGCT CAACGAGCAA
900

AGATACTACG GAGGTGGCTA TGGCTCCACG CAGGCTACCT TCATGGTATT CCAAGCCTTG
960

GCTCAATACC AAACAGATGT CCCTGACCAC AAGGACTTGA ACATGGATGT GTCCCTGCAC
1020

CTCCCCAGCC GC
1032


NUMBER OF SEQUENCE: 3
LENGTH OF SEQUENCE: 348
TYPE OF SEQUENCE:  amino acid
TOPOLOGY: linear
MOLECULE TYPE:  protein
FRAGMENT TYPE:  middle fragment
SEQUENCE:


Glu Gly Val Gln Lys Glu Asp Ile Pro Pro Ala Asp Leu Ser Asp
 1               5                   10                  15

Gln Val Pro Asp Thr Glu Ser Glu Thr Arg Ile Leu Leu Gln Gly
                 20                  25                  30

Thr Pro Val Ala Gln Met Thr Glu Asp Ala Val Asp Ala Glu Arg
                 35                  40                  45

Leu Lys His Leu Ile Val Thr Pro Ser Gly Cys Gly Glu Gln Asn
                 50                  55

Met Ile Gly Met Thr Pro Thr Val Ile Ala Val His Tyr Leu Asp
65 70 75

Glu Thr Glu Gln Trp Glu Lys Phe Gly Leu Glu Lys Arg Gln Gly
80 85 90

Ala Leu Glu Leu Ile Lys Lys Gly Tyr Thr Gln Gln Leu Ala Phe
95 100 105

Arg Gln Pro Ser Ser Ala Phe Ala Ala Phe Val Lys Arg Ala Pro
110 115 120

Ser Thr Trp Leu Thr Ala Tyr Val Val Lys Val Phe Ser Leu Ala
125 130 135

Val Asn Leu Ile Ala Ile Asp Ser Gln Val Leu Cys Gly Ala Val
140 145 150

Lys Trp Leu Ile Leu Glu Lys Gln Lys Pro Asp Gly Val Phe Gln
155 160 165

Glu Asp Ala Pro Val Ile His Gln Glu Met Ile Gly Gly Leu Arg
170 175 180

Asn Asn Asn Glu Lys Asp Met Ala Leu Thr Ala Phe Val Leu Ile
185 190 195

Ser Leu Gln Glu Ala Lys Asp Ile Cys Glu Glu Gln Val Asp Ser
200 205 210

Leu Pro Gly Ser Ile Thr Lys Ala Gly Asp Phe Leu Glu Ala Asn
215 220 225

Tyr Met Asn Leu Gln Arg Ser Tyr Thr Val Ala Ile Ala Gly Tyr
230 235 240

Ala Leu Ala Gln Met Gly Arg Leu Lys Gly Pro Leu Leu Asn Lys
245 250 255

Phe Leu Thr Thr Ala Lys Asp Lys Asn Arg Trp Glu Asp Pro Gly
260 265 270

Lys Gln Leu Tyr Asn Val Glu Ala Thr Ser Tyr Ala Leu Leu Ala
275 280 285

Leu Leu Gln Leu Lys Asp Phe Asp Phe Val Pro Pro Val Val Arg
290 295 300

Trp Leu Asn Glu Gln Arg Tyr Tyr Gly Gly Gly Tyr Gly Ser Thr
305 310 315

Gln Ala Thr Phe Met Val Phe Gln Ala Leu Ala Gln Tyr Gln Lys
320 325 330

16

EP 0 495 995 A1

Asp Ala Pro Asp His Gln Glu Leu Asn Leu Asp Val Ser Leu Gln
                335                 340                 345

Leu Pro Ser Arg

NUMBER OF SEQUENCE:  4

LENGTH OF SEQUENCE: 1047

TYPE OF SEQUENCE:  nucleic acid

STRAND TYPE:  double

TOPOLOGY:  linear

MOLECULE TYPE:  cDNA to mRNA

FRAGMENT TYPE:  middle fragment

IMMEDIATE SOURCE

        Library Name:  human cDNA clone pHLC3.11

        Clone Name:  pTHC3/1.050

SEQUENCE:

```
GAAGGAGTGC AGAAAGAGGA CATCCCACCT GCAGACCTCA GTGACCAAGT CCCGGACACC
GAGTCTGAGA CCAGAATTCT CCTGCAAGGG ACCCCAGTGG CCCAGATGAC AGAGGATGCC
GTCGACGCGG AACGGCTGAA GCACCTCATT GTGACCCCCT CGGGCTGCGG GGAACAGAAC
ATGATCGGCA TGACGCCCAC GGTCATCGCT GTGCATTACC TGGATGAAAC GGAGCAGTGG
GAGAAGTTCG GCCTAGAGAA GCGGCAGGGG GCCTTGGAGC TCATCAAGAA GGGGTACACC
CAGCAGCTGG CCTTCAGACA ACCCAGCTCT GCCTTTGCGG CCTTCGTGAA ACGGGCACCC
AGCACCTGGC TGACCGCCTA CGTGGTCAAG GTCTTCTCTC TGGCTGTCAA CCTCATCGCC
ATCGACTCCC AAGTCCTCTG CGGGGCTGTT AAATGGCTGA TCCTGGAGAA GCAGAAGCCC
GACGGGGTCT TCCAGGAGGA TGCGCCCGTG ATACACCAAG AAATGATTGG TGGATTACGG
AACAACAACG AGAAAGACAT GGCCCTCACG GCCTTTGTTC TCATCTCGCT GCAGGAGGCT
AAAGATATTT GCGAGGAGCA GGTCAACAGC CTGCCAGGCA GCATCACTAA AGCAGGAGAC
TTCCTTGAAG CCAACTACAT GAACCTACAG AGATCCTACA CTGTGGCCAT TGCTGGCTAT
GCTCTGGCCC AGATGGGCAG GCTGAAGGGG CCTCTTCTTA ACAAATTTCT GACCACAGCC
AAAGATAAGA ACCGCTGGGA GGACCCTGGT AAGCAGCTCT ACAACGTGGA GGCCACATCC
TATGCCCTCT TGGCCCTACT GCAGCTAAAA GACTTTGACT TTGTGCCTCC CGTCGTGCGT
TGGCTCAATG AACAGAGATA CTACGGTGGT GGCTATGGCT CTACCCAGGC CACCTTCATG
GTGTTCCAAG CCTTGGCTCA ATACCAAAAG GACGCCCCTG ACCACCAGGA ACTGAACCTT
GATGTGTCCC TCCAACTGCC CAGCCGC 1047
```

## Claims

1. A plasmid comprising a DNA sequence encoding an inhibitory protein for phospholipase $A_2$ originated from inflammatory sites and a DNA sequence having a function for an expression regulation thereof.

17

2. A plasmid in accordance with claim 1, wherein said DNA sequence encoding the inhibitory protein originates from rat or human.

3. A plasmid in accordance with claim 1, wherein said DNA sequence encoding the inhibitory protein is a DNA sequence encoding a protein constructing the amino acid sequence represented as sequence number 1 in the "Sequence Listing", or an amino acid sequence wherein parts of amino acids residues are mutually replaced in the sequence, or one or more of said amino acid residues are deleted from or newly inserted to the sequence.

4. A plasmid in accordance with claim 1, wherein said DNA sequence encoding the inhibitory protein is a DNA sequence encoding a protein constructing the amino acid sequence represented as seqeuence number 3 in the "Sequence Listing", or an amino acid sequence wherein parts of said amino acid residues are mutually replaced in the sequence, or one or more of said amino acid residues are deleted from or newly inserted to the sequence.

5. A plasmid in accordance with claim 3, wherein said DNA sequence encoding the inhibitory protein is a DNA sequence represented as sequence number 2 in the "Sequence Listing",

6. A plasmid in accordance with claim 4, wherein said DNA sequence encoding the inhibitory protein is a DNA sequence represented as sequence number 4 in the "Sequence Listing".

7. A plasmid in accordance with claim 5, wherein said DNA encoding the inhibitory protein comprises 2.1 kbp of a DNA fragment originated from rat liver cDNA λgt II library, and
said DNA fragment is reconstructed using 2.0 kbp of a DNA fragment prepared by digesting the cDNA λgt library with EcoRI and 0.1 kbp of a DNA fragment prepared by digesting another 0.7 kbp of a DNA fragment from the cDNA λgt library with BamHI.

8. A plasmid in accordance with claim 1, wherein said DNA sequence is reconstructed by ligation of a double-stranded DNA prepared by regating a 5' terminal 59 bp of a rat C3dg gene to a downstream region in an initiation codon, and to an upstream region in 1.0 kbp of EcoRI-FoKI fragment obtained by a digestion of 2.0 kbp of DNA fragment in accordance with claim 7 with FoKI,
following by ligation of a double-stranded DNA prepared by adding a 3' terminal 14 bp of rat C3dg gene to an upstream region in a termination codon, and to a downstream region in the 1.0 kbp EcoRI-FokI fragment.

9. A plasmid in accordance with claim 1, wherein said DNA sequence is reconstructed by ligation of a NcoI-EcoRI fragment of double-stranded DNA prepared by ligating with an initiation codon and 5' terminal 70 bp of human C3dg gene, to an upstream region in an about 1.2 kbp EcoRI-KpnI fragment obtained by a digestion of a human cDNA clone pHLC3.11 with EcoRI and KpnI,
following by a ligation of a EcoT14I-KpnI fragment of double-stranded DNA prepared by ligating with a 3' terminal 80 bp of a human C3dg gene and termination codon, to a downstream region of an about 0.97 kb NcoI-EcoT14I fragment obtained by a partial digestion of the resulting DNA fragment according to the previous ligation with EcoT14I.

10. A plasmid in accordance with claim 1, wherein said plasmid is selected from group consisting of ppTC3/2.1, pTRc3/1.035 and pTHC3/1.050.

11. A recombinant microbial cell transformed with a plasmid in accordance with claim 1.

12. A recombinant microbial cell in accordance with claim 11, wherein a host cell is a microbial cell belonging to a genus of Eschericia.

13. A recombinant microbial cell in accordance with claim 11, wherein the host cell is Eschericia coli JM109.

14. A recombinant microbial cell in accordance with claim 13, wherein the microbial cell is selected from a group consisting of Eshericia coli pTC3/2.1, J9THC 3/1.035 and J9THC3/1.050.

15. A method of producing a phospholipase $A_2$ inhibitory protein from inflammatory sites, characterised in that the recombinant microbial cells in accordance with claim 11 are cultured in a nutrient medium up to an accumulation of the inhibitory protein for phospholipase $A_2$ originated from inflammatory sites, and the inhibitory protein for phospholipase $A_2$ originated from inflammatory sites is recovered from the cultures.

16. A method in accordance with claim 15, wherein said recombinant microbial cell is as described in claim 14.

17. An inhibitory protein for phospholipase $A_2$ originated from inflammatory sites produced based on the method as described in claim 15.

18. An inhibitory protein for phospholipase $A_2$ originated from inflammatory sites in accordance with claim 17, produced based on the method as described in claim 16.

19. A pharmaceutical composition for suppression of allergic reaction comprising an inhibitory protein for phospholipase $A_2$ originated from inflammatory sites as an active ingredient.

20. A pharmaceutical composition for suppression of allergic reactions in accordance with claim 19, wherein said phospholipase $A_2$ inhibitory protein is as described in claim 17.

21. A pharmaceutical composition in accordance with claim 19, wherein said allergic reaction is a delayed type allergic reaction.

# Fig.1

# Fig.2a

(Continue to Fig. 2b)

# F i g.2b

ligation

EcoRI
NcoI
FokI
BanⅡ
rat C3dg gene
pTRC3/1.035

# Fig.3a

# Fig.3b

double stranded synthetic DNA
(about 80bp)

Termination Codon

# Fig.4a

# Fig.4b

# Fig.5

# Fig.6

## INTERNATIONAL SEARCH REPORT

International Application No  PCT/JP91/01040

| I. CLASSIFICATION OF SUBJECT MATTER (if several classification symbols apply, indicate all) [6] |
|---|

According to International Patent Classification (IPC) or to both National Classification and IPC

Int. Cl$^5$  C12N15/12, 15/72, 1/21, 9/99, C12P21/02, C07K13/00, A61K37/64 (C12N1/21, C12R1:19), (C12P21/02, C12R1:19)

### II. FIELDS SEARCHED

Minimum Documentation Searched [7]

| Classification System | Classification Symbols |
|---|---|
| IPC. | C12N15/00-15/90, C12N1/21, C12N9/99, C12P21/00-21/02, C07K13/00, A61K37/64 |

Documentation Searched other than Minimum Documentation
to the Extent that such Documents are Included in the Fields Searched [8]

Biosis Database, Chemical Abstracts Database (CA, REG), EMBL-GDB, GenBank, NBRF-PDB, Swiss-Prot

### III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X/Y | Proc. Natl. Acad. Sci. USA, Vol.87, No.7, 1990, Suwa Y. et al. "Protein ace ous inhibitors of phospholipase A$_2$ purified from inflammatory sites in rats" P.2395-2399 | 17-21/1-4, 11-13, 15 |
| Y | The Journal of Biological chemistry, Vol.259, No.22, 1984, Wetsel et al. "Structure Murine Complement Component C3"  P.13857-13862 | 1-4, 11-13, 15 |
| Y | Proc. Natl. Acad. Sci. USA, Vol.82, No.3, 1985, Bruijn M. et al. "Human complement component C3:cDNA coding sequence and derived primary structure"  P.708-712 | 1-4, 11-13, 15 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

### IV. CERTIFICATION

| Date of the Actual Completion of the International Search | Date of Mailing of this International Search Report |
|---|---|
| October 24, 1991 (24. 10. 91) | November 11, 1991 (11. 11. 91) |

| International Searching Authority | Signature of Authorized Officer |
|---|---|
| Japanese Patent Office | |